# FASCICULE DE BREVET EUROPEEN

(11) **EP 1 415 645 B1**
(45) Date de publication et mention de la délivrance du brevet: **03.10.2007**
(21) Numéro de dépôt: 03292410.2
(22) Date de dépôt: 30.09.2003
(51) Int. Cl.: A61K 8/368, A61K 8/44, A61K 8/81, A61K 8/892, A61Q 19/00

(54) **Composition sous forme d'émulsion huile-dans-eau et ses utilisations notamment cosmétiques**
Zusammensetzung in Form einer Öl-in-Wasser Emulsion und deren kosmetische Verwendungen
Composition in the form of an oil-in-water emulsion and its cosmetic uses

(30) Priorité: 29.10.2002 FR 0213520; 29.10.2002 FR 0213521
(43) Date de publication de la demande: 06.05.2004
(60) Demande divisionnaire: 07101299.1
(73) Titulaire: L'ORÉAL, 75008 Paris (FR)
(72) Inventeur: Noel, Christine, 94260 Fresnes (FR); Livernette, Anne-France, 75012 Paris (FR)
(74) Mandataire: Rasson, Catherine

(56) Documents cités:
- EP-A- 0 739 619
- EP-A- 0 970 690
- EP-A- 1 055 406
- EP-A- 1 172 077
- EP-A- 1 243 250
- WO-A-02/03952
- FR-A- 2 771 633

## Description

La présente demande concerne une composition notamment cosmétique, sous forme d'émulsion huile-dans-eau contenant un organopolysiloxane élastomère, un polymère hydrophile et un dérivé lipophile d'acide salicylique. La demande se rapporte aussi à l'utilisation de ladite composition notamment dans le domaine cosmétique, et à l'utilisation du dérivé d'acide salicylique pour la stabilisation d'une émulsion huile-dans-eau contenant un polymère hydrophile et un organopolysiloxane élastomère.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme phase externe, une phase aqueuse, ce qui leur confère, lors de l'application sur la peau, un toucher plus frais, moins gras et plus léger que les émulsions E/H.

Les émulsions sont généralement stabilisées par des tensioactifs émulsionnants appropriés qui, grâce à leur structure amphiphile, se placent à l'interface huile/eau et stabilisent ainsi les gouttelettes dispersées. Ces émulsionnants présentent cependant l'inconvénient d'être pénétrants et potentiellement irritants pour la peau, les yeux et le cuir chevelu, notamment pour les sujets à peau sensible.

En outre, de telles émulsions peuvent avoir des propriétés cosmétiques et physico-chimiques insuffisantes (toucher huileux, instabilité dans le temps). Le fait d'augmenter le taux des tensioactifs ne résout pas généralement les problèmes mentionnés. La stabilité requise n'est pas toujours atteinte et les propriétés cosmétiques ne sont pas améliorées (toucher cireux, lourd, manque de fraîcheur à l'application). Par ailleurs, comme indiqué ci-dessus, il est aussi déconseillé d'utiliser un trop fort taux de tensioactif pour des raisons d'innocuité.

Une solution pour s'affranchir des phénomènes d'instabilité des émulsions H/E (crémage et déphasage) consiste à introduire dans l'émulsion, des agents épaississants dont la fonction est de créer, au sein de la phase aqueuse, une matrice gélifiée servant à figer les gouttelettes huileuses et assurant un maintien mécanique de l'ensemble de l'émulsion. Par ailleurs, il a été envisagé de remplacer les tensioactifs par des polymères hydrophiles comportant dans leur chaîne une partie hydrophile et une partie hydrophobe, tels que les copolymères d'alkyl-C₁₀-C₃₀-acrylate et d'acide acrylique ou méthacrylique, comme le produit "PEMULEN TR2" commercialisé par la société Goodrich, ou par des polymères hydrophiles dérivés d'acide 2-acrylamido 2-méthylpropane sulfonique (AMPS) comme décrit dans le document EP-A-815844.

Toutefois, les émulsions stabilisées par des polymères hydrophiles peuvent avoir un toucher rêche. Pour améliorer la douceur de ces émulsions; la demanderesse a cherché à y incorporer des composés apportant de la douceur, et notamment des organopolysiloxanes élastomères (appelés aussi élastomères de silicone), tels que les produits commercialisés par Shin-Etsu sous les dénominations KSG. Cependant, la demanderesse a constaté que, quand on introduit ces organopolysiloxanes élastomères en grande quantité, et notamment en une quantité supérieure à 1 % (en matière active), ils ont tendance à déstabiliser l'émulsion contenant le polymère hydrophile.

L'objectif de l'invention est de pouvoir réaliser des émulsions huile-dans-eau (H/E) et présentant de bonnes propriétés cosmétiques (douceur), qui soient stables, c'est-à-dire qui ne se déphasent ni ne présentent de relargage d'huile, et ce quelle que soit la quantité d'élastomère de silicone contenue dans l'émulsion.

La demanderesse a découvert de façon inattendue que l'utilisation d'un dérivé lipophile d'acide salicylique permettait de réaliser des émulsions huile-dans-eau contenant à la fois un polymère hydrophile et un élastomère de silicone, qui soient stables, cette stabilité persistant même quand le taux d'élastomère de silicone est important. En outre, selon le polymère utilisé et en particulier quand le polymère hydrophile est un polymère d'AMPS, on peut préparer des émulsions stables bien qu'éventuellement exemptes de tensioactif classiquement utilisé dans ce type d'émulsion.

Ainsi, la présente invention se rapporte à une composition pour application topique sous forme d'émulsion huile-dans-eau comprenant une phase huileuse dispersée dans une phase aqueuse, et un polymère hydrophile, caractérisée par le fait qu'elle contient (1) au moins un organopolysiloxane élastomère et (2) un dérivé lipophile choisi parmi les dérivés lipophiles d'acide salicylique de formule de formule (1) suivante ou un sel d'un tel dérivé : 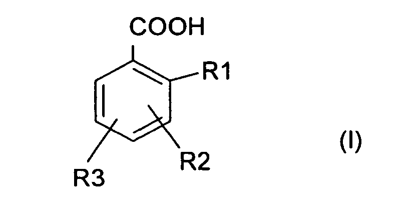 dans laquelle :
- R₁ représente un radical hydroxyle ou un ester de formule :

   -O-CO-R₄

   dans laquelle R₄ est un radical aliphatique, saturé ou insaturé, comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone,
- R₂ et R₃ indépendamment l'un de l'autre se trouvent en position 3, 4, 5 ou 6 sur le noyau benzénique et représentent indépendamment l'un de l'autre, un atome d'hydrogène ou un radical :

   -(O)ₙ-(CO)ₘ-R₅

   dans lequel n et m, indépendamment l'un de l'autre, sont chacun un nombre entier égal à 0 ou 1 ; à la condition que R₂ et R₃ ne soient pas simultanément des atomes d'hydrogène ;
- R₅ représente un hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone, un radical aromatique comprenant de 6 à 10 atomes de carbone.

Le dérivé lipophile utilisé dans la composition selon l'invention permet d'obtenir une émulsion stable. L'invention a donc aussi pour objet l'utilisation d'un dérivé lipophile choisi parmi les dérivés lipophiles d'acide salicylique de formule (1), pour stabiliser une émulsion huile-dans-eau contenant un organopolysiloxane élastomère et un polymère hydrophile.

La composition étant destinée à une application topique, elle contient un milieu physiologiquement acceptable. On entend par "milieu physiologiquement acceptable", un milieu non toxique et susceptible d'être appliqué sur la peau (y compris l'intérieur des paupières), les lèvres, les ongles ou les cheveux d'êtres humains.

### Dérivé lipophile d'acide salicylique

Le dérivé lipophile d'acide salicylique utilisé dans la composition de l'invention est un composé de formule (1).

De manière préférée, le dérivé d'acide salicylique de formule (1) est tel que R₁ représente un radical hydroxyle, R₂ représente un atome d'hydrogène, R₃ est en position 5 du noyau benzénique et R₅ représente un radical aliphatique saturé comprenant de 3 à 15 atomes de carbone.

Selon un mode de réalisation préféré de l'invention, le dérivé d'acide salicylique de formule (1) est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents et leurs mélanges. Il s'agit plus particulièrement de l'acide n-octanoyl-5-salicylique (nom INCl : Capryloyl salicylic Acid).

On peut utiliser un ou plusieurs dérivés lipophiles d'acide salicylique. La quantité de dérivé(s) lipophile(s) peut aller par exemple de 0,01 à 20 % et de préférence de 0,05 à 10 % en poids et mieux de 0,1 à 5 % en poids par rapport au poids total de la composition.

### Organopolysiloxane élastomère

La composition de l'invention contient au moins un organopolysiloxane élastomère, de préférence au moins partiellement réticulé. On entend par « élastomère » un matériau solide souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères utilisés dans la composition selon l'invention sont de préférence partiellement ou totalement réticulés. Ils se présentent sous forme de particules. En particulier, les particules d'organopolysiloxane élastomère ont une taille allant de 0,1 à 500 µm, de préférence de 3 à 200 µm et mieux de 3 à 50 µm. Ces particules peuvent avoir toute forme et par exemple être sphériques, plates ou amorphes.

Quand ils sont inclus dans une phase huileuse, ces organopolysiloxanes élastomères se transforment, selon le taux de phase huileuse utilisé, en un produit d'aspect spongieux lorsqu'ils sont utilisés en présence de faibles teneurs en phase huileuse, ou en un gel homogène en présence de quantités de phase huileuse plus élevées. La gélification de la phase huileuse par ces élastomères peut être totale ou partielle.

Ainsi les élastomères de l'invention peuvent être véhiculés sous forme de gel anhydre constitué d'un organopolysiloxane élastomère et d'une phase huileuse. La phase huileuse utilisée lors de la fabrication du gel anhydre d'organopolysiloxane élastomère contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Les organopolysiloxanes élastomères utilisés selon l'invention peuvent être choisis parmi les polymères réticulés décrits dans la demande EP-A-0295886 et parmi ceux décrits dans le brevet US-A-5,266,321.

Ce sont de préférence des organopolysiloxanes élastomères obtenus par réaction d'addition et de réticulation, en présence d'un catalyseur, de préférence un catalyseur du type platine, d'au moins :
- (a) un organopolysiloxane ayant deux groupements vinyliques en position α-ω de la chaîne siliconée par molécule; et
- (b) un organopolysiloxane ayant au moins deux atomes d'hydrogène liés à un atome de silicium par molécule.

Le premier organopolysiloxane (i) est choisi parmi les polydiméthylsiloxanes ; il s'agit de préférence d' un α-ω-diméthylvinyl polydiméthylsiloxane.

L'organopolysiloxane est de préférence dans un gel obtenu selon les étapes suivantes :
- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur, de préférence d'un catalyseur de platine.

Les organopolysiloxanes élastomères utilisés dans la composition de l'invention peuvent être par exemple ceux commercialisés sous les noms KSG 6 par la société Shin-Etsu ; Trefil E-505C ou Trefil E-506C par la société Dow-Corning ; Gransil (SR-CYC, SR DMF10, SR-DC556) par la société Grant Industries, ou ceux commercialisés sous forme de gels déjà constitués : KSG 15, KSG 16, KSG 17, KSG 18, KSG 26A, KSG 26B, de la société Shin-Etsu ; Gransil SR 5CYC gel, Gransil SR DMF 10 gel, Gransil SR DC556 gel de la société Grant Industries ; 1229-02-167 et 1229-02-168 de la société General Electric. On peut aussi utiliser un mélange d'élastomères de silicone, et notamment un mélange de ces produits commerciaux.

De manière préférée, l'organopolysiloxane élastomère utilisé dans la composition de l'invention se présente sous forme d'un gel anhydre, et notamment d'un gel anhydre formé de particules non sphériques d'organopolysiloxane élastomère, tels que les KSG. L'organopolysiloxane élastomère est préférentiellement introduit dans la phase huileuse de l'émulsion selon l'invention.

Le ou les organopolysiloxanes élastomères utilisés selon l'invention sont présents en une quantité en matière active qui varie selon le but recherché. Cette quantité peut aller par exemple de 0,5 à 20 %, de préférence de 1 à 15 % et mieux de 5 à 10 % du poids total de la composition.

### Polymères hydrophiles

Les polymères hydrophiles sont des polymères hydrosolubles ou hydrodispersibles. Par "polymère hydrosoluble ou hydrodispersible", on entend un polymère qui, introduit dans de l'eau à une concentration égale à 1 %, conduit à une solution macroscopiquement homogène dont la transmittance de la lumière, à une longueur d'onde égale à 500 nm, à travers un échantillon de 1 cm d'épaisseur, est d'au moins 10%.

Ces polymères sont des agents gélifiants, et ils peuvent être choisis en particulier parmi les polymères carboxyvinyliques; les copolymères acryliques ou méthacryliques ; les gommes naturelles ; les polysaccharides ; les polymères (homopolymères et copolymères) d'acrylamide ; et leurs mélanges. Ces polymères peuvent se présenter tels quels ou sous forme de latex (en dispersions).

Comme polymères carboxyvinyliques, on peut citer par exemple les polymères d'acide acrylique réticulés (nom INCI : Carbomer), tels que les produits vendus sous les noms Carbopols 980, 981, 954, 2984 et 5984 par la société NOVEON ou les produits vendus sous les noms Synthalen M et Synthalen K par la société 3 VSA.

Comme copolymères acryliques ou méthacryliques, on peut citer notamment les copolymères d'acrylates d'alkyle en C₁₀-C₃₀ et d'acide acrylique ou méthacrylique ou de leur ester, vendus sous les dénominations Pemulen TR1, Pemulen TR2, Carbopol 1342 par la société NOVEON (nom INCI: Acrylates/C10-30 Alkyl Acrylate Crosspolymer).

Comme gommes naturelles, on peut citer par exemple la gomme de xanthane, la gomme de gellane, la gomme de caroube.

Comme polysaccharides, on peut citer notamment les dérivés de cellulose, tels que par exemple l'hydroxypropylmethylcellulose, la carboxyméthylcellulose.

Comme polymères d'acrylamide, on peut citer notamment les acides poly(méth)acrylamido-alkyl(C₁-C₄)-sulfoniques. Ces polymères sont de préférence réticulés, et, en outre, ils sont de préférence partiellement ou totalement neutralisés.

Parmi ces polymères, on peut citer notamment :
- l'acide polyacrylamido-méthane-sulfonique,
- l'acide polyacrylamido-éthane-sulfonique,
- l'acide polyacrylamido-propane-sulfonique,
- l'acide poly-2-acrylamido-2-méthylpropane-sulfonique,
- l'acide poly-2-méthacrylamido-2-méthylpropane-sulfonique,
- l'acide poly-2-acrylamido-n-butane-sulfonique.

Des polymères de ce type et notamment des acides poly-2-acrylamido-2-méthylpropane-sulfoniques réticulés et partiellement ou totalement neutralisés sont connus, décrits et préparés dans le document DE-A-19625810.

Les acides poly(méth)acrylamido-alkyl(C₁-C₄)-sulfoniques préférés sont réticulés et neutralisés à au moins 90%. Ces polymères peuvent être réticulés notamment par un motif réticulant ayant au moins deux doubles-liaisons oléfiniques. Les motifs réticulants ayant au moins deux doubles-liaisons oléfiniques peuvent être choisis par exemple parmi le dipropylèneglycol-diallyléther, les polyglycol-diallyléthers, le triéthylèneglycol-divinyléther, l'hydroquinone-diallyl-éther, le tétra-allyl-oxyéthane ou d'autres allyl- ou vinyl-éthers d'alcools polyfonctionnels, le diacrylate de tétraéthylèneglycol, la triallylamine, le triméthylolpropane-diallyléther, le méthylène-bis-acrylamide ou le divinylbenzène.

Les motifs réticulants ayant au moins deux doubles-liaisons oléfiniques sont plus particulièrement encore choisis parmi ceux répondant à la formule générale (III) suivante : 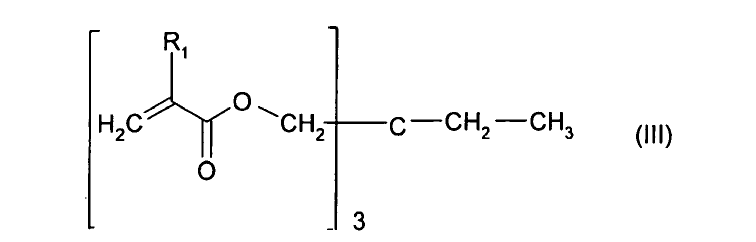 dans laquelle R₁ désigne un atome d'hydrogène ou un radical alkyle en C₁-C₄. Le motif réticulant peut être plus particulièrement le triméthylol propane triacrylate (R₁ = méthyle).

Les acides poly(méth)acrylamido-alkyl(C₁-C₄)-sulfoniques préférés sont notamment les acides poly-2-acrylamido-2-méthylpropane-sulfoniques qui sont caractérisés par le fait qu'ils comprennent, distribués de façon aléatoire :
a) de 90 à 99,9% en poids de motifs de formule (IV) suivante : 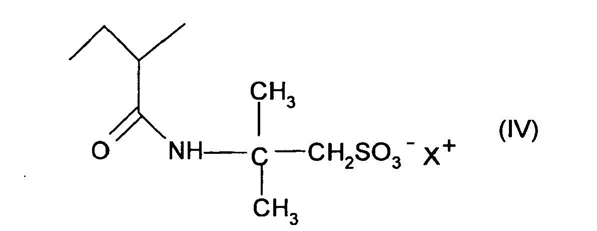 dans laquelle X⁺ désigne un cation ou un mélange de cations, dont H⁺ ,
b) de 0,01 à 10% en poids d'au moins un motif réticulant ayant au moins deux doubles-liaisons oléfiniques,
les proportions en poids étant définies par rapport au poids total du polymère.

X⁺ représente un cation ou un mélange de cations, choisis en particulier parmi un proton (H+), un cation de métal alcalin, un cation équivalent de celui d'un métal alcalino-terreux ou l'ion ammonium.

De préférence, l'acide poly-2-acrylamido-2-méthylpropane-sulfonique réticulé et neutralisé utilisé comporte de 98 à 99,5 % en poids de motifs de formule (III) et de 0,5 à 2 % en poids de motifs réticulants, le motif réticulant étant de préférence le triméthylol propane triacrylate.

Les acides poly-2-acrylamido-2-méthylpropane-sulfoniques réticulés et partiellement ou totalement neutralisés sont généralement connus sous les appellations "Ammonium Polycrylamido-2-methylpropanesulfonate" ou encore "Ammonium Polyacryldimethyltauramide" (appellation INCI).

Un produit particulièrement préféré selon l'invention est celui vendu par la société CLARIANT sous la dénomination commerciale HOSTACERIN AMPS, qui est un acide poly-2-acrylamido-2-méthylpropane sulfonique réticulé et partiellement neutralisé par l'ammoniaque.

Les acides poly(méth)acrylamido-alkyl(C₁-C₄)-sulfoniques réticulés peuvent être obtenus selon le procédé de préparation connu comprenant les étapes suivantes :
(a) on disperse ou on dissout le monomère acide 2-(méth)acrylamido-alkyl(C₁-C₄)- sulfonique sous forme libre dans une solution de tertio-butanol ou dans une solution d'eau et de tertio-butanol ;
(b) on neutralise la solution ou la dispersion de monomère obtenue en (a) par une ou plusieurs bases minérales ou organiques, de préférence l'ammoniaque, dans une quantité permettant d'obtenir un taux de neutralisation des fonctions acides sulfoniques du polymère allant de 0 à 100% ;
(c) on ajoute à la solution ou dispersion obtenue en (b), le ou les monomères réticulants ;
(d) on effectue une polymérisation radicalaire classique en présence d'amorceurs de radicaux libres à une température allant de 10 à 150°C, le polymère précipitant dans la solution ou la dispersion à base de tertio-butanol.

Comme polymères d'acrylamide, on peut citer aussi le copolymère réticulé d'acrylamide et d'acide 2-acrylamido 2-méthylpropane sulfonique, en particulier le mélange vendu sous le nom Sepigel 305 par la Société SEPPIC qui se présente sous forme d'une émulsion à environ 40 % de copolymère (nom INCI: polyacrylamide/C 13-14 Isoparaffin/laureth-7).

De manière préférée, le polymère hydrophile utilisé dans la composition de l'invention est introduit dans la phase aqueuse de l'émulsion selon l'invention.

Selon un mode préféré de réalisation de l'invention, le polymère hydrophile est un acide poly-2-acrylamido-2-méthylpropane-sulfonique réticulé et partiellement ou totalement neutralisé, en particulier le sel d'ammonium d'un tel acide.

La quantité en matière active de polymère(s) hydrophile(s) va de préférence de 0,1 à 10 % en poids, de préférence de 0,2 à 5 % en poids et mieux de 0,5 à 2 % en poids par rapport au poids total de la composition.

### Phase huileuse

Outre les huiles éventuellement présentes dans le gel d'organopolysiloxane élastomère, la phase huileuse peut être de toute nature et comprendre des huiles, des cires ou des gommes solides à température ambiante, des corps gras pâteux, d'origine animale, végétale, minérale ou synthétique et leurs mélanges.

Comme huiles utilisables dans la composition de l'invention, on peut citer notamment :
- les huiles hydrocarbonées d'origine animale, telles que le perhydrosqualène ;
- les huiles hydrocarbonées d'origine végétale, telles que les triglycérides liquides d'acides gras, par exemple les huiles de tournesol, de maïs, de soja, de courge, de coriandre, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools ;
- les hydrocarbures linéaires ou ramifiés, d'origine minérale ou synthétique, tels que les huiles de paraffine volatiles ou non et leurs dérivés, la vaseline, les polydécènes, le polyisobutène hydrogéné tel que l'huile de Parléam® ;
- les éthers de synthèse de formule R³OR⁴ dans laquelle R³ est un radical alkyle en C₃ à C₁₉ et R⁴ un radical alkyle en C₃ à C₂₀ ;
- des alcools gras comme l'octyldodécanol ou l'alcool oléique ;
- les huiles fluorées partiellement hydrocarbonées et/ou siliconées comme les perfluoropolyesters ;
- les huiles de silicone comme les polyméthylsiloxanes à chaîne siliconée linéaire ou cyclique, liquides ou pâteux à température ambiante, les phényldiméthicones, les phényltriméthicones, les polyméthylphénylsiloxanes, les alkylpolydiméthyl-siloxanes avec une chaîne alkyle en C₂ à C₂₀ ;
- leurs mélanges.

Selon un mode préféré de réalisation de l'invention, la phase huileuse comprend au moins une huile volatile. Par huile volatile, on entend en particulier une huile susceptible de s'évaporer, en moins d'une heure, au contact de la peau ou des lèvres, ayant notamment une pression vapeur non nulle, en particulier allant de 10⁻³ à 300 mm de Hg (à température ambiante et pression atmosphérique) et de préférence supérieure à 0,3 mm de Hg. Comme huiles volatiles, on peut citer notamment les huiles de silicone volatiles, telles que polyméthylsiloxanes à chaîne siliconée linéaire ou cyclique, et notamment les huiles de silicone cyclomethicones comme le cyclopentasiloxane, le cyclohexasiloxane, le cyclotétrasiloxane et leurs mélanges.

La quantité de phase huileuse dans la composition de l'invention peut aller de 1 à 50 % en poids, de préférence de 5 à 40 % et mieux de 10 à 30 % en poids par rapport au poids total de la composition.

### Phase aqueuse

La quantité de phase aqueuse dans la composition de l'invention peut aller de 50 à 99 % en poids, de préférence de 60 à 95 % et mieux de 70 à 90 % en poids par rapport au poids total de la composition.

La phase aqueuse comprend au moins de l'eau. Elle peut comprendre en outre un ou plusieurs solvants hydrosolubles. Comme solvants hydrosolubles, on peut citer par exemple les mono-alcools linéaires ou ramifiés ayant de 1 à 8 atomes de carbone, comme l'éthanol, le propanol, le butanol, l'isopropanol, l'isobutanol ; les polyéthylène glycols ayant de 6 à 80 oxydes d'éthylène ; les polyols comme le propylène glycol, la glycérine, l'isoprène glycol et le butylène glycol.

Selon un mode préférée de réalisation de l'invention, l'émulsion de l'invention est exempte de tensioactif classiquement utilisé dans les H/E et elle présente de ce fait l'avantage de ne pas être irritante pour les peaux particulièrement sensibles. En outre, cette émulsion présente l'avantage de permettre l'incorporation d'actifs thermosensibles car elle peut être fabriquée à température ambiante.

### Adjuvants

De façon connue, les compositions de l'invention peuvent contenir des adjuvants habituels dans les domaines considérés. Comme adjuvants, on peut citer par exemple les actifs, les conservateurs, les antioxydants, les agents complexants, les ajusteurs de pH (acides ou basiques), les parfums, les bactéricides, les absorbeurs d'odeur, les charges, les matières colorantes (pigments ou colorants), et encore les vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme actifs utilisables dans la composition de l'invention, on peut citer par exemple, les enzymes (par exemple lactoperoxydase, lipase, protéase, phospholipase, cellulases) ; les flavonoïdes ; les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) ; les agents matifiants comme les fibres ; les agents tenseurs ; les céramides ; les huiles essentielles ; et leurs mélanges ; et tout actif approprié pour le but final de la composition.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les documents US-A-4367390, EP-A-863145, EP-A-517104, EP-A-570838, EP-A-796851, EP-A-775698, EP-A-878469, EP-A-933376, EP-A-507691, EP-A-507692, EP-A-790243, EP-A-944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les documents EP-A-669323 et US-A-2,463,264; les dérivés de l'acide p-aminobenzoïque (PABA) ; les dérivés de méthylène bis-(hydroxyphényl benzotriazole) tels que décrits dans les documents US-A-5,237,071, US-A-5,166,355, GB-A-2303549, DE-A-19726184 et EP-A-893119 ; les polymères filtres et silicones filtres tels que ceux décrits notamment dans le document WO-A-93/04665 ; les dimères dérivés d'α-alkylstyrène tels que ceux décrits dans le document DE-A-19855649 ; les 4,4-diarylbutadiènes tels que décrits dans les documents EP-A-967200, DE-A-19746654, DE-A-19755649, EP-A-1008586, EP-A-1133980 et EP-A-133981, et leurs mélanges.

Les agents photoprotecteurs organiques plus particulièrement préférés sont choisis parmi les composés suivants :
- Ethylhexyl Salicylate vendu sous le nom commercial NEO HELIOPAN OS par HAARMANN et REIMER ;
- Ethylhexyl Methoxycinnamate vendu notamment sous le nom commercial PARSOL MCX par HOFFMANN LA ROCHE ;
- Octocrylene (α-cyano-β,β-diphénylacrylate de 2-éthylhexyle) vendu notamment sous le nom commercial UVINUL N539 par BASF ;
- Phenylbenzimidazole Sulfonic Acid,
- Benzophenone-3 ou Oxybenzone, vendue sous le nom commercial UVINUL M40 par BASF ;
- Benzophenone-4 vendue sous le nom commercial UVINUL MS40 par BASF;
- 4-Methylbenzylidene camphor vendu sous le nom commercial EUSOLEX 6300 par MERCK ;
- Terephthalylidene Dicamphor Sulfonic Acid fabriqué sous le nom MEXORYL SX par CHIMEX ;
- Disodium Phenyl Dibenzimidazole Tetra-sulfonate ;
- la 2,4,6-tris-(4'-amino benzalmalonate de diisobutyle)-s-triazine ;
- Anisotriazine vendu sous le nom commercial TINOSORB S par CIBA GEIGY ;
- le Butyl méthoxydibenzoylmethane vendu notamment sous le nom commercial PARSOL 1789 par HOFFMANN LA ROCHE ;
- et leurs mélanges.

Comme agents photoprotecteurs inorganiques (ou filtres physiques), on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Comme charges, on peut citer par exemple, les particules de polyamide (Nylon) et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; la silice ; les oxydes métalliques tels que le dioxyde de titane ou l'oxyde de zinc ; le mica ; les fibres telles que les fibres de Nylon 6 (ou Polyamide 6) et de Nylon 6,6 (ou Polyamide 66), et leurs mélanges. La quantité de charge(s) peut aller par exemple de 0,05 à 20 % en poids et mieux 0,1 à 10 % en poids par rapport au poids total de la composition.

La composition de l'invention est utilisée en application topique, et elle peut en particulier constituer une composition cosmétique ou dermatologique. Cette composition peut être plus ou moins fluide et avoir l'aspect d'une crème blanche ou colorée, d'une pommade, d'un lait, d'une lotion, d'un sérum, d'une pâte, d'une mousse. La composition de l'invention peut être appliquée par voie topique, sur le visage, y compris autour des yeux, sur le corps ainsi que sur le cuir chevelu des êtres humains.

La composition, objet de l'invention, trouve son application notamment dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres (avec incorporation de pigments et/ou de colorants) et/ou pour la protection solaire (avec incorporation d'agents photoprotecteurs). Elle peut être destinée en particulier à lutter contre les signes du vieillissement cutané comme composition anti-âge pour la peau, et notamment pour améliorer l'éclat du teint de la peau. Elle peut être utilisée dans toute autre application, notamment pour la peau, appropriée au but recherché selon les actifs présents dans la composition.

Aussi, l'invention a encore pour objet l'utilisation cosmétique de la composition cosmétique telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition cosmétique telle que définie ci-dessus.

L'invention a aussi pour objet l'utilisation cosmétique d'une composition cosmétique telle que définie ci-dessus, pour lutter contre les signes du vieillissement de la peau et/ou pour améliorer l'éclat du teint de la peau.

Les exemples qui suivent permettront de mieux comprendre l'invention, sans toutefois présenter un caractère limitatif. Les quantités indiquées sont en % en poids, sauf mention contraire.

### Exemple 1 selon l'invention

### Phase aqueuse :

- Hostacerin AMPS (vendu par la société Hoechst) 2 %
- Conservateurs 0,4 %
- colorant 0,8 %
- Eau déminéralisée qsp 100 %

### Phase huileuse :

- Cyclopentasiloxane 6 %
- KSG 16 (à 24 % de matière active)
   5 % (soit 1,2 % de matière active)
- Acide n-octanoyl-5-salicylique 0,01 %

Mode opératoire : On chauffe l'eau, les conservateurs, et les colorants à 75/80°C. On y disperse l'AMPS sous agitation jusqu'à l'obtention d'un gel transparent et lisse. On refroidit à 55°C, puis on ajoute sous agitation le cyclopentasiloxane, et l'acide n-octanoyl-5-salicylique. On refroidit vers 40°C, et on ajoute le KSG-16 sous agitation, puis on refroidit à température ambiante.

On obtient une crème lisse, très douce sur la peau. Au microscope, les globules de KSG sont bien dispersés, et la crème est régulière. Cette crème est apte à améliorer l'éclat du teint de la peau tout en étant très douce.

### Exemple comparatif 1

On réalise une composition identique à celle de l'exemple 1 mais ne contenant pas d'acide n-octanoyl-5-salicylique. On obtient une émulsion peu lisse, qui présente au microscope des globules importants de KSG.

### Exemple 2 selon l'invention

### Phase aqueuse :

- Hostacerin AMPS (vendu par la société Hoechst) 2 %
- Conservateurs 0,4 %
- colorant 0,8 %
- Eau déminéralisée qsp 100 %

### Phase huileuse :

- Cyclopentasiloxane 6 %
- KSG 16 (à 24 % de matière active)
   15 % (soit 3,6 % de matière active)
- Acide n-octanoyl-5-salicylique 0,01 %

Le mode opératoire est analogue à celui de l'exemple 1.

On obtient une crème lisse et très douce sur la peau. Au microscope, les globules de KSG sont assez bien dispersés, et la crème est régulière. Cette crème est apte à améliorer l'éclat du teint de la peau tout en étant très douce.

### Exemple comparatif 2

On réalise une composition identique à celle de l'exemple 2 mais ne contenant pas d'acide n-octanoyl-5-salicylique. On obtient une émulsion granuleuse, qui présente au microscope de grosses plages de KSG et des dépôts de KSG sur les bords de l'émulsion.

## Revendications

1. Composition pour application topique sous forme d'émulsion huile-dans-eau comprenant une phase huileuse dispersée dans une phase aqueuse, et un polymère hydrophile, **caractérisée par le fait qu'**elle contient (1) au moins un organopolysiloxane élastomère et (2) au moins un dérivé lipophile choisi parmi les dérivés lipophiles d'acide salicylique de formule (1) suivante ou un sel d'un tel composé : 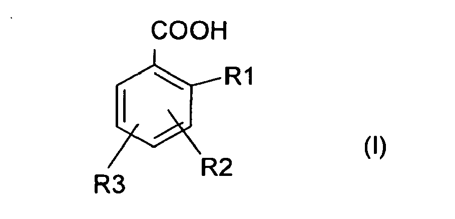 dans laquelle :
- R₁ représente un radical hydroxyle ou un ester de formule :
- O-CO-R₄
dans laquelle R₄ est un radical aliphatique, saturé ou insaturé, comprenant de 1 à 26 atomes de carbone, une fonction amine ou thiol éventuellement substituée par un radical alkyle comprenant de 1 à 18 atomes de carbone,
- R₂ et R₃ indépendamment l'un de l'autre se trouvent en position 3, 4, 5 ou 6 sur le noyau benzénique et représentent indépendamment l'un de l'autre un atome d'hydrogène ou un radical :
- (O)ₙ-(CO)ₘ-R₅
dans lequel n et m, indépendamment l'un de l'autre, sont chacun un nombre entier égal à 0 ou 1 ; à la condition que R₂ et R₃ ne soient pas simultanément des atomes d'hydrogène ;
- R₅ représente un hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone, un radical aromatique comprenant de 6 à 10 atomes de carbone.

2. Composition selon la revendication 1, **caractérisée en ce que** le dérivé d'acide salicylique de formule (1) est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents, et leurs mélanges.

3. Composition selon la revendication précédente, **caractérisée en ce que** le dérivé lipophile d'acide salicylique est l'acide n-octanoyl-5-salicylique.

4. Composition selon l'une quelconque des revendications précédentes, **caractérisée en ce que** la quantité de dérivé(s) lipophile(s) va de 0,01 à 20 % et de préférence de 0,05 à 10 % en poids par rapport au poids total de la composition.

5. Composition selon l'une quelconque des revendications précédentes, **caractérisée en ce que** l'organopolysiloxane élastomère est obtenu par réaction d'addition et de réticulation, en présence d'un catalyseur, d'au moins :
- un premier organopolysiloxane (i) ayant deux groupements vinyliques en position α-ω de la chaîne siliconée par molécule ; et
- un second organopolysiloxane (ii) ayant au moins un atome d'hydrogène lié à un atome de silicium par molécule.

6. Composition selon la revendication précédente, **caractérisée en ce que** le premier organopolysiloxane (i) est un **α-ω-**diméthylvinyl polydiméthylsiloxane.

7. Composition selon l'une quelconque des revendications précédentes, **caractérisée en ce que** l'organopolysiloxane est dans un gel obtenu selon les étapes suivantes :
- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur de platine.

8. Composition selon l'une des revendications précédentes, **caractérisée en ce que** la quantité d'organopolysiloxane(s) élastomère(s) va de 0,5 à 20 % en poids par rapport au poids total de la composition, et de préférence de 1 à 15 % en poids par rapport au poids total de la composition.

9. Composition selon l'une quelconque des revendications précédentes, **caractérisée en ce que** le polymère hydrophile est choisi parmi les polymères carboxyvinyliques ; les copolymères acryliques ou méthacryliques ; les gommes naturelles ; les polysaccharides ; les polymères d'acrylamide, et leurs mélanges.

10. Composition selon l'une quelconque des revendications précédentes, **caractérisée en ce que** le polymère hydrophile est un acide poly(méth)acrylamido-alkyl(C₁-C₄)-sulfonique.

11. Composition selon la revendication précédente, **caractérisée en ce que** l'acide poly(méth)acrylamido-alkyl(C₁-C₄)-sulfonique est réticulé et neutralisé à au moins 90%.

12. Composition selon la revendication 10 ou 11, **caractérisée en ce que** l'acide poly(méth)acrylamido-alkyl(C₁-C₄)-sulfonique est un acide polyacrylamido-méthyl-propanesulfonique comprenant, distribués de façon aléatoire :
a) de 90 à 99,9% en poids de motifs de formule générale (IV) suivante : 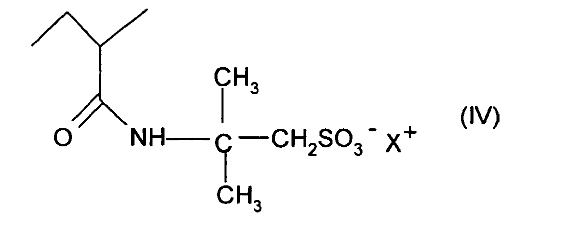 dans laquelle X⁺ désigne un cation ou un mélange de cations, dont H⁺ ,
b) de 0,01 à 10% en poids d'au moins un motif réticulant ayant au moins deux doubles-liaisons oléfiniques,
les proportions en poids étant définies par rapport au poids total du polymère.

13. Composition selon la revendication précédente, **caractérisée en ce que** l'acide polyacrylamido-méthylpropane-sulfonique comporte de 98 à 99,5 % en poids de motifs de formule (IV) et de 0,2 à 2 % en poids de motifs réticulants.

14. Composition selon l'une quelconque des revendications précédentes, **caractérisée en ce que** la quantité de polymère hydrophile va de 0,1 à 10 % en poids par rapport au poids total de la composition, et de préférence de 0,2 à 5 % en poids par rapport au poids total de la composition.

15. Composition selon l'une quelconque des revendications précédentes, **caractérisée en ce que** la quantité de phase huileuse va de 1 à 50 % en poids par rapport au poids total de la composition.

16. Composition selon l'une quelconque des revendications précédentes, **caractérisée en ce que** la phase huileuse comprend au moins une huile volatile.

17. Composition selon l'une quelconque des revendications précédentes, **caractérisée en ce qu'**elle est exempte de tensioactif.

18. Composition selon l'une quelconque des revendications précédentes, **caractérisée en ce qu'**elle constitue une composition cosmétique ou dermatologique.

19. Utilisation cosmétique d'une composition cosmétique selon l'une quelconque des revendications 1 à 17, pour le traitement, la protection, le soin, le démaquillage et/ou le nettoyage de la peau, des lèvres et/ou des cheveux, et/ou pour le maquillage de la peau et/ou des lèvres.

20. Procédé de traitement cosmétique de la peau, des cheveux, et/ou des lèvres, **caractérisé par le fait qu'**on applique sur la peau, les cheveux et/ou les lèvres, une composition cosmétique selon l'une quelconque des revendications 1 à 17.

21. Utilisation cosmétique d'une composition cosmétique selon l'une quelconque des revendications 1 à 17, pour lutter contre les signes du vieillissement de la peau et/ou pour améliorer l'éclat du teint de la peau.

22. Utilisation d'un dérivé lipophile choisi parmi les dérivés lipophiles d'acide salicylique de formule (1), pour stabiliser une émulsion huile-dans-eau contenant un organopolysiloxane élastomère et un polymère hydrophile.

## Claims

1. Composition for topical application in the form of an oil-in-water emulsion comprising an oily phase dispersed in an aqueous phase, and a hydrophilic polymer, **characterized in that** it contains (1) at least one elastomeric organopolysiloxane and (2) at least one lipophilic derivative chosen from lipophilic salicylic acid derivatives of formula (I) below or a salt of such a compound: 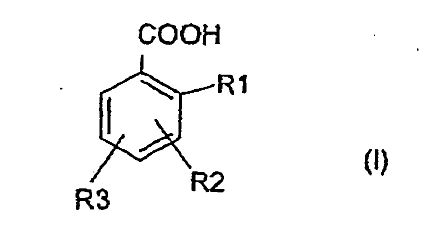 in which:
- R₁ represents a hydroxyl radical or an ester of formula:
-O-CO-R₄
in which R₄ is a saturated or unsaturated aliphatic radical containing from 1 to 26 carbon atoms, an amine or thiol function optionally substituted with an alkyl radical containing from 1 to 18 carbon atoms,
- R₂ and R₃, independently of each other, are in position 3, 4, 5 or 6 on the benzene ring and represent, independently of each other, a hydrogen atom or a radical:
-(O)ₙ-(CO)ₘ-R₅
in which n and m, independently of each other, are each an integer equal to 0 or 1; on condition that R₂ and R₃ are not simultaneously hydrogen atoms;
- R₅ represents a hydrogen, a linear, branched or cyclized saturated aliphatic radical containing from 1 to 18 carbon atoms, an unsaturated radical containing from 3 to 18 carbon atoms, bearing one to nine conjugated or non-conjugated double bonds, the radicals possibly being substituted with at least one substituent chosen from halogen atoms (fluorine, chlorine, bromine or iodine), trifluoromethyl radicals, hydroxyl in free form or esterified with an acid containing from 1 to 6 carbon atoms, or carboxyl in free form or esterified with a lower alcohol containing from 1 to 6 carbon atoms, or an aromatic radical containing from 6 to 10 carbon atoms.

2. Composition according to Claim 1, **characterized in that** the salicylic acid derivative of formula (I) is chosen from 5-n-octanoylsalicylic acid, 5-n-decanoylsalicylic acid, 5-n-dodecanoylsalicylic acid, 5-n-octylsalicylic acid, 5-n-heptyloxysalicylic acid, 4-n-heptyloxysalicylic acid, 5-tert-octylsaXicylic acid, 3-tert-butyl-5-methylsalicylic acid, 3-tert-butyl-6-methylsalicylic acid, 3,5-diisopropylsalicylic acid, 5-butoxysalicylic acid, 5-octyloxysalicylic acid, 5-propanoylsalicylic acid, 5-n-hexadecanoylsalicylic acid, 5-n-oleoylsalicylic acid, 5-benzoylsalicylic acid, monovalent and divalent salts thereof, and mixtures thereof.

3. Composition according to the preceding claim, **characterized in that** the lipophilic salicylic acid derivative is 5-n-octanoylsalicylic acid.

4. Composition according to any one of the preceding claims, **characterized in that** the amount of lipophilic derivative(s) ranges from 0.01% to 20% and preferably from 0.05% to 10% by weight relative to the total weight of the composition.

5. Composition according to any one of the preceding claims, **characterized in that** the elastomeric organopolysiloxane is obtained by addition and crosslinking reaction, in the presence of a catalyst, of at least:
- a first organopolysiloxane (i) containing two vinyl groups in α-ω position on the silicone chain per molecule; and
- a second organopolysiloxane (ii) containing at least one hydrogen atom linked to a silicon atom per molecule.

6. Composition according to the preceding claim, **characterized in that** the first organopolysiloxane (i) is an α,ω-dimethylvinylpolydimethylsiloxane.

7. Composition according to any one of the preceding claims, **characterized in that** the organopolysiloxane is in a gel obtained according to the following steps:
- (a) mixing of the first and second organopolysiloxanes (i) and (ii);
- (b) adding an oily phase to the mixture from step (a);
- (c) polymerizing the first and second organopolysiloxanes (i) and (ii) in the oily phase in the presence of a platinum catalyst.

8. Composition according to one of the preceding claims, **characterized in that** the amount of elastomeric organopolysiloxane(s) ranges from 0.5% to 20% by weight relative to the total weight of the composition, and preferably from 1% to 15% by weight relative to the total weight of the composition.

9. Composition according to any one of the preceding claims, **characterized in that** the hydrophilic polymer is chosen from carboxyvinyl polymers; acrylic or methacrylic copolymers; natural gums; polysaccharides; acrylamide polymers, and mixtures thereof.

10. Composition according to any one of the preceding claims, **characterized in that** the hydrophilic polymer is a poly(meth)acrylamido(C₁-C₄)alkylsulfonic acid.

11. Composition according to the preceding claim, **characterized in that** the poly(meth)acrylamido(C₁-C₄)alkylsulfonic acid is crosslinked and at least 90% neutralized.

12. Composition according to ; Claim 10 or 11, **characterized in that** the poly (meth) acrylamido (C₁-C₄) alkylsulfonic acid is a polyacrylamidomethylpropanesulfonic acid comprising, randomly distributed:
a) from 90% to 99.9% by weight of units of general formula (IV) below: 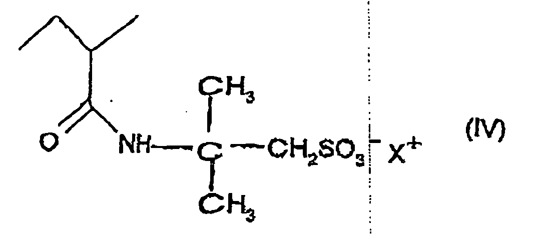 in which X⁺ denotes a cation or a mixture of cations, including H⁺,
b) from 0.01% to 10% by weight of at least one crosslinking unit containing at least two olefinic double bonds,
the weight proportions being defined relative to the total weight of the polymer.

13. Composition according to the preceding claim, **characterized in that** the polyacrylamidomethylpropanesulfonic acid comprises from 98% to 99.5% by weight of units of formula (IV) and from 0.2% to 2% by weight of crosslinking units.

14. Composition according to any one of the preceding claims, **characterized in that** the amount of hydrophilic polymer ranges from 0.1% to 10% by weight relative to the total weight of the composition, and preferably from 0.2% to 5% by weight relative to the total weight of the composition.

15. Composition according to any one of the preceding claims, **characterized in that** the amount of oily phase ranges from 1% to 50% by weight relative to the total weight of the composition.

16. Composition according to any one of the preceding claims, **characterized in that** the oily phase comprises at least one volatile oil.

17. Composition according to any one of the preceding claims, **characterized in that** it is free of surfactant.

18. Composition according to any one of the preceding claims, **characterized in that** it constitutes a cosmetic or dermatological composition.

19. Cosmetic use of a cosmetic composition according to any one of Claims 1 to 17, for treating, protecting, caring for, removing makeup from and/or cleansing the skin, the lips and/or the hair, and/or for making up the skin and/or the lips.

20. Cosmetic process for treating the skin, the hair and/or the lips, **characterized in that** a cosmetic composition according to any one of Claims 1 to 17 is applied to the skin, the hair and/or the lips.

21. Cosmetic use of a cosmetic composition according to any one of Claims 1 to 17, to combat the signs of ageing of the skin and/or to improve the radiance of the complexion of the skin.

22. Use of a lipophilic derivative chosen from lipophilic salicylic acid derivatives of formula (I), to stabilize an oil-in-water emulsion containing an elastomeric organopolysiloxane and a hydrophilic polymer.

## Patentansprüche

1. Zusammensetzung für die topische Anwendung in Form einer Öl-in-Wasser-Emulsion, die eine in einer wässrigen Phase dispergierte Ölphase und ein hydrophiles Polymer enthält, **dadurch gekennzeichnet, dass** sie (1) mindestens ein elastomeres Organopolysiloxan und (2) mindestens ein lipophiles Derivat enthält, das unter den lipophilen Salicylsäurederivaten der folgenden Formel (I) oder einem Salz einer solchen Verbindung ausgewählt ist: 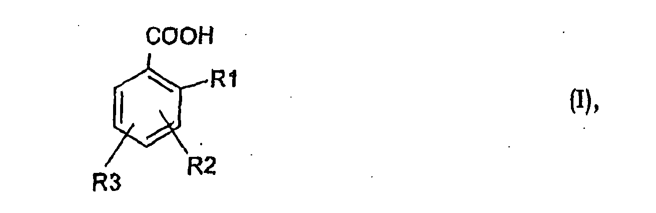 wobei in der Formel bedeuten:
- R₁ bedeutet eine Hydroxygruppe oder einen Ester der Formel:
-O-CO-R₄
worin R₄ eine gesättigte oder ungesättigte, aliphatische Gruppe mit 1 bis 26 Kohlenstoffatomen oder eine Amin- oder Thiolfunktion bedeutet, die gegebenenfalls mit einer Alkylgruppe mit 1 bis 18 Kohlenstoffatomen substituiert ist,
- R₂ und R₃ befinden sich unabhängig voneinander in 3-, 4-, 5-oder 6-Stellung des Benzolrings und bedeuten unabhängig voneinander ein Wasserstoffatom oder eine Gruppe
-(O)ₙ-(CO)ₘ-R₅
worin n und m unabhängig voneinander jeweils 0 oder 1 bedeuten; mit der Maßgabe, dass die Gruppen R₂ und R₃ nicht gleichzeitig Wasserstoff bedeuten;
- R₅ bedeutet Wasserstoff, eine gesättigte aliphatische Gruppe mit 1 bis 18 Kohlenstoffatomen, die geradkettig, verzweigt oder cyclisiert ist, eine ungesättigte Gruppe mit 3 bis 18 Kohlenstoffatomen, die eine bis neun Doppelbindungen aufweist, die konjugiert oder nicht konjugiert sind, wobei die Gruppen mit mindestens einem Substituenten substituiert sein können, der unter den Halogenatomen (Fluor, Chlor, Brom, Iod), Trifluormethyl, Hydroxygruppen in freier Form oder verestert mit einer Säure mit 1 bis 6 Kohlenstoffatomen oder Carboxygruppen in freier Form oder verestert mit einem niederen Alkohol mit 1 bis 6 Kohlenstoffatomen ausgewählt ist, eine aromatische Gruppe mit 6 bis 10 Kohlenstoffatomen.

2. Zusammensetzung nach Anspruch 1, **dadurch gekennzeichnet, dass** das Salicylsäurederivat der Formel (1) unter 5-*n*-Octanoylsalicylsäure, 5-*n*-Decanoylsalicylsäure, 5-*n*-Dodecanoylsalicylsäure, 5-*n*-Octylsalicylsäure, 5-*n*-Heptyloxysalicylsäure, 5-*n-*Heptyloxysalicylsäure, 5-*t*-Octylsahcylsäure, 3-*t*-Butyl-5-methylsalicylsäure, 3-*t*-Butyl-6-methylsalicylsäure, 3,5-Diisopropylsalicylsäure, 5-Butoxysalicylsäure, 5-Octyloxysalicylsäure, 5-Propanoylsalicylsäure, 5-*n*-Hexadecanoylsalicylsäure, 5-*n-*Oleoylsalicylsäure, 5-Benzoylsalicylsäure, deren einwertigen und zweiwertigen Salzen und deren Gemischen ausgewählt ist.

3. Zusammensetzung nach dem vorhergehenden Anspruch, **dadurch gekennzeichnet, dass** es sich bei dem lipophilen Salicylsäurederivat um 5-*n*-Octanoylsalicylsäure handelt.

4. Zusammensetzung nach einem der vorhergehenden Ansprüche, **dadurch gekennzeichnet, dass** der Mengenanteil des lipophilen Derivats (der lipophilen Derivate) im Bereich von 0,01 bis 20 Gew.-% und vorzugsweise 0,05 bis 10 Gew.-%, bezogen auf das Gesamtgewicht der Zusammensetzung, liegt.

5. Zusammensetzung nach einem der vorhergehenden Ansprüche, **dadurch gekennzeichnet, dass** das elastomere Organopolysiloxan durch Addition und Vernetzung von zumindest den, folgenden Verbindungen in Gegenwart eines Katalysators gebildet wird:
- eines ersten Organopolysiloxans (i) mit zwei Vinylgruppen in α-ω-Stellung der Siliconkette pro Molekül; und
- eines zweiten Organopolysiloxans (ii) mit mindestens einem Wasserstoffatom, das an ein Siliciumatom gebunden ist, pro Molekül.

6. Zusammensetzung nach dem vorhergehenden Anspruch, **dadurch gekennzeichnet, dass** das erste Organopolysiloxan (i) ein α-ω- Dimethylvinylpolydimethylsiloxan ist.

7. Zusammensetzung nach einem der vorhergehenden Ansprüche, **dadurch gekennzeichnet, dass** das Organopolysiloxan in einem Gel vorliegt, das nach den folgenden Schritten hergestellt wird:
- (a) Mischen des ersten und des zweiten Organopolysiloxans (i) und (ii);
- (b) Zugabe einer Ölphase zu dem Gemisch aus Schritt (a);
- (c) Polymerisation des ersten und zweiten Organopolysiloxans (i) und (ii) in der Ölphase in Gegenwart eines Platinkatalysators.

8. Zusammensetzung nach einem der vorhergehenden Ansprüche, **dadurch gekennzeichnet, dass** der Mengenanteil des elastomeren Organopolysiloxans (der elastomeren Organopolysiloxane) im Bereich von 0,5 bis 20 Gew.-%, bezogen auf das Gesamtgewicht der Zusammensetzung, und vorzugsweise 1 bis 15 Gew.-%, bezogen auf das Gesamtgewicht der Zusammensetzung, liegt.

9. Zusammensetzung nach einem der vorhergehenden Ansprüche, **dadurch gekennzeichnet, dass** das hydrophile Polymer unter den Carboxyvinylpolymeren; Acryl- oder Methacrylcopolymeren; natürlichen Gummen; Polysacchariden; Acrylpolymeren und deren Gemischen ausgewählt ist.

10. Zusammensetzung nach einem der vorhergehenden Ansprüche, **dadurch gekennzeichnet, dass** das hydrophile Polymer eine Poly(meth)acrylamidoalkyl(C₁₋₄)sulfonsäure ist.

11. Zusammensetzung nach dem vorhergehenden Anspruch, **dadurch gekennzeichnet, dass** die Poly(meth)acrylamidoalkyl(C₁₋₄)-sulfonsäure zu mindestens 90 % neutralisiert und vernetzt ist.

12. Zusammensetzung nach Anspruch 10 oder 11, **dadurch gekennzeichnet, dass** die Poly(meth)acrylamidoalkyl(C₁₋₄)sulfonsäure eine Polyacrylamidomethylpropansulfonsäure ist, die zufällig verteilt umfasst:
a) 90 bis 99,9 Gew.-% Einheiten der folgenden allgemeinen Formel (IV): 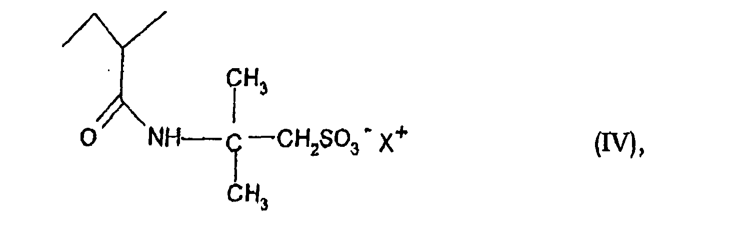 worin X⁺ ein Kation oder ein Gemisch von Kationen, darunter H⁺, bedeutet,
b) 0,01 bis 10 Gew.-% mindestens einer Vernetzungseinheit,
die mindestens zwei olefinische Doppelbindungen aufweist, wobei die Gewichtsanteile bezogen auf das Gesamtgewicht des Polymers definiert sind.

13. Zusammensetzung nach dem vorhergehenden Anspruch, **dadurch gekennzeichnet, dass** die Polyacrylamidomcthylpropansulfonsäure 98 bis 99,5 Gew.-% Einheiten der Formel (IV) und 0,2 bis 2 Gew.-% Vernetzungseinheiten aufweist.

14. Zusammensetzung nach einem der vorhergehenden Ansprüche, **dadurch gekennzeichnet, dass** der Mengenanteil des hydrophilen Polymers im Bereich von 0,1 bis 10 Gew.-%, bezogen auf das Gesamtgewicht der Zusammensetzung, und vorzugsweise 0,2 bis 5 Gew.-%, bezogen auf das Gesamtgewicht der Zusammensetzung, liegt.

15. Zusammensetzung nach einem der vorhergehenden Ansprüche, **dadurch gekennzeichnet, dass** der Mengenanteil der Ölphase im Bereich von 1 bis 50 Gew.-%, bezogen auf das Gesamtgewicht der Zusammensetzung, liegt.

16. Zusammensetzung nach einem der vorhergehenden Ansprüche, **dadurch gekennzeichnet, dass** die Ölphase mindestens ein flüchtiges Öl enthält.

17. Zusammensetzung nach einem der vorhergehenden Ansprüche, **dadurch gekennzeichnet, dass** sie keinen grenzflächenaktiven Stoff enthält.

18. Zusammensetzung nach einem der vorhergehenden Ansprüche, **dadurch gekennzeichnet, dass** es sich um eine kosmetische oder dermatologische Zusammensetzung handelt.

19. Verwendung einer kosmetischen Zusammensetzung nach einem der Ansprüche 1 bis 17 zur Behandlung, zum Schutz, zur Pflege, zum Abschminken und/oder zur Reinigung der Haut, der Lippen und/ oder der Haare und/ oder zum Schminken der Haut und/oder der Lippen.

20. Verfahren zur kosmetischen Behandlung der Haut, der Haare und/oder der Lippen, **dadurch gekennzeichnet, dass** auf die Haut, die Haare und/oder die Lippen eine kosmetische Zusammensetzung nach einem der Ansprüche 1 bis 17 aufgetragen wird.

21. Kosmetische Verwendung einer kosmetischen Zusammensetzung nach einem der Ansprüche 1 bis 17 zur Bekämpfung der Anzeichen der Hautalterung und/oder um die Ausstrahlung des Teints zu verbessern.

22. Verwendung eines lipophilen Derivats, das unter den lipophilen Salicylsäurederivaten der Formel (I) ausgewählt ist, um eine Öl-in-Wasser-Emulsion zu stabilisieren, die ein elastomeres Organopolysiloxan und ein hydrophiles Polymer enthält.
